# EUROPEAN PATENT APPLICATION

(11) **EP 2 992 920 A1**
(43) Date of publication of application: **09.03.2016**
(21) Application number: 15171578.6
(22) Date of filing: 11.06.2015
(51) Int. Cl.: A61M 16/08

(54) **RESPIRATORY DEVICE HAVING FUNCTION OF DETECTING AIRFLOW PRESSURE DIFFERENCE**

(30) Priority: 05.09.2014 TW 103130712
(71) Applicant: Delta Electronics, Inc., Taoyuan Hsien 333 (TW)
(72) Inventor: LIN, Jung-Yu, 320 Taoyuan Hsien (TW); PAI, Sheng-Wen, 320 Taoyuan Hsien (TW); CHEN, Chun-Chen, 320 Taoyuan Hsien (TW); CHIEN, Shih-Kai, 320 Taoyuan Hsien (TW)
(74) Representative: 2K Patentanwälte Blasberg Kewitz & Reichel

(57) **Abstract**

A respiratory device (1) includes a host (11), a pressure sensor (116) and a connecting module (12). The host (11) includes a first airflow channel (112) and a first shell (111). The first shell (111) includes a communication part (113) running through the first shell (111). The pressure sensor (116) is installed in the host (11) and connected with the communication part (113). The connecting module (12) includes a second airflow channel (121), a filter (122) and an airflow guiding part (123). The second airflow channel (121) is in communication with the first airflow channel (112). The filter (122) is used for filtering an airflow (130) from a breathing tube (13). A first end of the airflow guiding part (123) is in communication with the communication part (113), and the unfiltered airflow (130) is introduced into a second end of the airflow guiding part (123). A pressure level of the unfiltered airflow (130) is detected by the pressure sensor (116) through the communication part (113) and the airflow guiding part (123).

## Description

### FIELD OF THE INVENTION

The present invention relates to a respiratory device, and more particularly to a respiratory device having a function of detecting an airflow pressure difference.

### BACKGROUND OF THE INVENTION

A continuous positive airway pressure (CPAP) respiratory device is widely used in a medical instrument to treat sleep apnea. The use of the CPAP respiratory device may maintain a continuous level of positive airway pressure of the user in order to avoid breathing obstruction.

Generally, the CPAP respiratory device is connected with a mask of the user through a breathing tube. Moreover, a pressure sensor is installed in the CPAP respiratory device to detect the breathing status of the user. That is, the respiratory airflow pressure generated by the user may be transmitted to the CPAP respiratory device through the breathing tube and detected by the pressure sensor. According to the detecting result of the pressure sensor, the CPAP respiratory device provides accurate and continuous pressure level to the user. Generally, the respiratory droplets produced by the user may be in direct contact with the CPAP respiratory device through the breathing tube. For blocking the respiratory droplets of the user from being introduced into the CPAP respiratory device through the breathing tube, a filter is usually installed in an airflow channel between the CPAP respiratory device and the breathing tube. Since the filter can avoid contamination of the CPAP respiratory device, the CPAP respiratory device can be reused.

However, since the filter is usually installed in an airflow channel between the CPAP respiratory device and the breathing tube, the airflow pressure detected by the pressure sensor of the CPAP respiratory device is substantially equal to the pressure of the respiratory airflow that is produced by the user and filtered by the filter. In fact, because of the installation of the filter, the airflow pressure before the filter and the airflow pressure after the filter are different. Since the detecting result of the pressure sensor is affected by the filter, the detecting result of the pressure sensor cannot actually reflect the respiratory airflow pressure produced by the user. In other words, the conventional CPAP respiratory device cannot precisely control the pressure level to the user.

Moreover, another CPAP respiratory device comprises a flow resistor and a flow sensor in addition to the pressure sensor. When an airflow passes through the flow resistor, a pressure difference between an inlet and an outlet of the flow resistor is generated. The pressure before the airflow enters the inlet of the flow resistor and the pressure after the airflow exits the outlet of the flow resistor are measured by the flow sensor. According to the pressure difference between the inlet and the outlet of the flow resistor, the flow sensor calculates a flow rate of the airflow. However, the installation of the flow resistor may increase the fabricating cost of the CPAP respiratory device.

Therefore, there is a need of providing a respiratory device having a function of detecting an airflow pressure difference in order to overcome the above drawbacks.

### SUMMARY OF THE INVENTION

The above problems are solved by a respiratory device according to claims 1, 8, 9 and 12. Further advantageous embodiments are the subject-matter of the dependent claims.

An object of the present invention provides a respiratory device for detecting a pressure level of an unfiltered airflow.

Another object of the present invention provides a respiratory device having a function of detecting an airflow pressure difference without the need of using a flow resistor so as to reduce the cost.

In accordance with an aspect of the present invention, there is provided a respiratory device. The respiratory device is in communication with a breathing tube. The respiratory device includes a host, a pressure sensor and a connecting module. The host includes a first airflow channel and a first shell. The first shell includes a communication part. The communication part runs through the first shell. The pressure sensor is installed in the host and connected with the communication part. The connecting module is detachably connected between the host and the breathing tube, and includes a second airflow channel, a filter and an airflow guiding part. The second airflow channel is in communication with the first airflow channel. The filter is installed in the second airflow channel. When the host and the connecting module are combined together, a first end of the airflow guiding part is in communication with the communication part, and a first portion of an airflow unfiltered by the filter is introduced into a second end of the airflow guiding part. A second portion of the airflow is introduced into the host through the second airflow channel, the filter and the first airflow channel. A pressure level of the unfiltered airflow is detected by the pressure sensor through the communication part and the airflow guiding part.

In accordance with another aspect of the present invention, there is provided a respiratory device. The respiratory device is in communication with a breathing tube. The respiratory device includes a host, a flow sensor and a connecting module. The host includes a first airflow channel and a first shell. The first shell includes a first communication part and a second communication part. The first communication part and the second communication part run through the first shell. The flow sensor is installed in the host, and connected with the first communication part and the second communication part. The connecting module is detachably connected between the host and the breathing tube, and includes a second airflow channel, a filter, a first airflow guiding part and a second airflow guiding part. The second airflow channel is in communication with the first airflow channel. The filter is installed in the second airflow channel. When the host and the connecting module are combined together, a first end of the first airflow guiding part is in communication with the first communication part, a first end of the second airflow guiding part is in communication with the second communication part, a first portion of an airflow unfiltered by the filter is introduced into a second end of the first airflow guiding part, and a second portion of the airflow filtered by the filter is introduced into a second end of the second airflow guiding part. A third portion of the airflow is introduced into the host through the second airflow channel, the filter and the first airflow channel. A first pressure level of the unfiltered airflow is detected by the flow sensor through the first communication part and the first airflow guiding part. A second pressure level of the filtered airflow is detected by the flow sensor through the second communication part and the second airflow guiding part. Consequently, a pressure difference between the first pressure level and the second pressure level is obtained.

In accordance with another aspect of the present invention, there is provided a respiratory device. The respiratory device is in communication with a breathing tube. The respiratory device includes a host, a flow sensor, a pressure sensor and a connecting module. The host includes a first airflow channel and a first shell. The first shell includes a first communication part and a second communication part. The first communication part and the second communication part run through the first shell. The flow sensor is installed in the host, and connected with the first communication part and the second communication part. The pressure sensor is installed in the host and connected with the first communication part. The connecting module is detachably connected between the host and the breathing tube, and includes a second airflow channel, a filter, a first airflow guiding part and a second airflow guiding part. The second airflow channel is in communication with the first airflow channel. The filter is installed in the second airflow channel. When the host and the connecting module are combined together, a first end of the first airflow guiding part is in communication with the first communication part, a first end of the second airflow guiding part is in communication with the second communication part, a first portion of an airflow unfiltered by the filter is introduced into a second end of the first airflow guiding part, and a second portion of the airflow filtered by the filter is introduced into a second end of the second airflow guiding part. A third portion of the airflow is introduced into the host through the second airflow channel, the filter and the first airflow channel. A first pressure level of the unfiltered airflow is detected by both of the flow sensor and the pressure sensor through the first communication part and the first airflow guiding part. A second pressure level of the filtered airflow is detected by the flow sensor through the second communication part and the second airflow guiding part. In addition, a pressure difference between the first pressure level and the second pressure level is obtained by the flow sensor.

In accordance with another aspect of the present invention, there is provided a respiratory device. The respiratory device is in communication with a breathing tube. The respiratory device includes a host, a pressure sensor and a connecting module. The host includes a first airflow channel and a first shell. The first shell includes a communication part. The communication part runs through the first shell. The pressure sensor is installed in the host and connected with the communication part. The connecting module is detachably connected between the host and the breathing tube, and includes a second airflow channel, a filter and an airflow guiding part. The second airflow channel is in communication with the first airflow channel. The airflow guiding part is arranged between the filter and the breathing tube. The filter is installed in the second airflow channel. When the host and the connecting module are combined together, the communication part is in communication with the airflow guiding part so as to receive an airflow from the breathing tube.

In accordance with another aspect of the present invention, there is provided a respiratory device. The respiratory device is in communication with a breathing tube. The respiratory device includes a host, a flow sensor and a connecting module. The host includes a first airflow channel and a first shell. The first shell includes a first communication part and a second communication part. The first communication part and the second communication part run through the first shell. The flow sensor is installed in the host, and connected with the first communication part and the second communication part. The connecting module is detachably connected between the host and the breathing tube, and includes a second airflow channel, a filter, a first airflow guiding part and a second airflow guiding part. The second airflow channel is in communication with the first airflow channel. The filter is installed in the second airflow channel. The first airflow guiding part is arranged between the filter and the breathing tube. When the host and the connecting module are combined together, the first airflow guiding part is in communication with the first communication part, the second airflow guiding part is in communication with the second communication part, and the first airflow guiding part and the second airflow guiding part receive an airflow from the breathing tube and provide the airflow to the flow sensor, respectively, so that a pressure difference of the airflow is obtained.

The above contents of the present invention will become more readily apparent to those ordinarily skilled in the art after reviewing the following detailed description and accompanying drawings, in which:

### BRIEF DESCRIPTION OF THE DRAWINGS

FIG. 1 is a schematic exploded view illustrating the relationship between a respiratory device and a breathing tube according to a first embodiment of the present invention;
FIG. 2 is a schematic assembled view illustrating the relationship between the respiratory device and the breathing tube of FIG. 1;
FIG. 3 is a schematic exploded view illustrating the relationship between a respiratory device and a breathing tube according to a second embodiment of the present invention;
FIG. 4 is a schematic assembled view illustrating the relationship between the respiratory device and the breathing tube of FIG. 3; and
FIG. 5 is a schematic exploded view illustrating the relationship between a respiratory device and a breathing tube according to a third embodiment of the present invention.

### DETAILED DESCRIPTION OF THE PREFERRED EMBODIMENT

The present invention will now be described more specifically with reference to the following embodiments. It is to be noted that the following descriptions of preferred embodiments of this invention are presented herein for purpose of illustration and description only. It is not intended to be exhaustive or to be limited to the precise form disclosed.

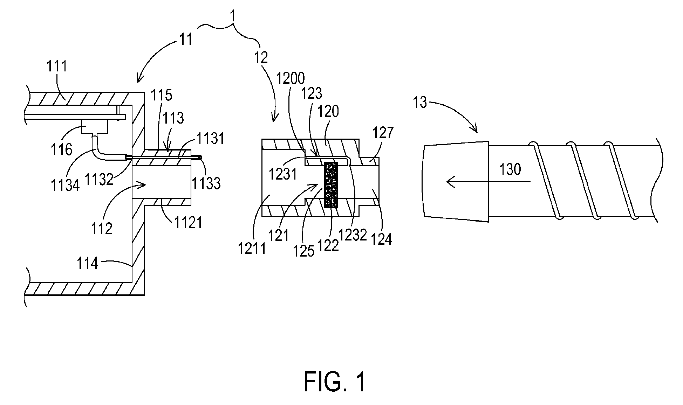

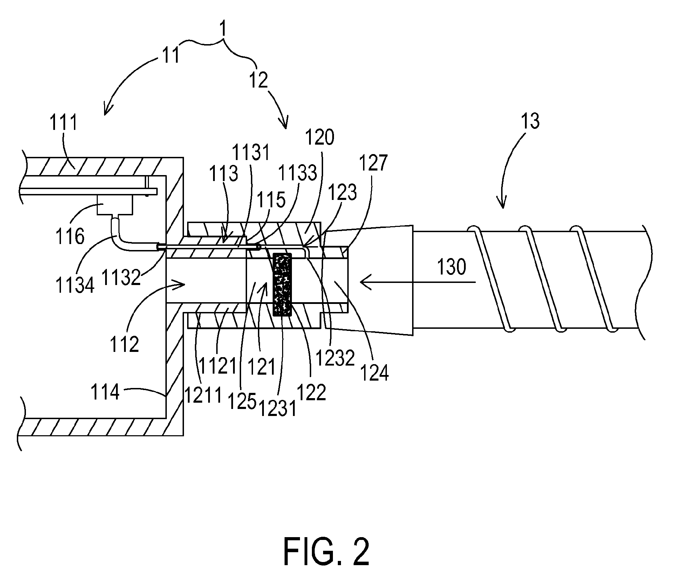

FIG. 1 is a schematic exploded view illustrating the relationship between a respiratory device and a breathing tube according to a first embodiment of the present invention. FIG. 2 is a schematic assembled view illustrating the relationship between the respiratory device and the breathing tube of FIG. 1. In this embodiment, the respiratory device 1 is a CPAP respiratory device, but is not limited thereto. The respiratory device 1 is detachably connected with the breathing tube 13. After the respiratory device 1 is connected with the breathing tube 13, the respiratory device 1 and the breathing tube 13 are in communication with each other. The breathing tube 13 may receive an airflow 130, which is blown by the user. Consequently, the airflow 130 may be transmitted to the respiratory device 1 through the breathing tube 13. According to the airflow 130, the respiratory device 1 performs the corresponding actions. In this embodiment, the respiratory device 1 comprises a host 11, a connecting module 12 and a pressure sensor 116. The host 11 is used for controlling the actions of the respiratory device 1. The host 11 comprises a first shell 111 and a first airflow channel 112. The first shell 111 comprises one communication part 113, an inner wall 114 and an outer wall 115. The inner wall 114 and the outer wall 115 are opposed to each other. The communication part 113 runs through the first shell 111. Consequently, the inner wall 114 and the outer wall 115 are connected with each other through the communication part 113. The first airflow channel 112 is defined by at least one part of the first shell 111.

In this embodiment, the communication part 113 comprises a hollow channel 1131, a first hollow post 1132 and a second hollow post 1133. The hollow channel 1131 runs through the first shell 111, and is arranged between the inner wall 114 and the outer wall 115 of the first shell 111. A first end and a second end of the hollow channel 1131 are connected with the inner wall 114 and the outer wall 115 of the first shell 111, respectively. The first hollow post 1132 is a hollow structure protruded from the inner wall 114 of the first shell 111. In addition, the first hollow post 1132 is connected with the first end of the hollow channel 1131. The second hollow post 1133 is a hollow structure protruded from the outer wall 115 of the first shell 111. In addition, the second hollow post 1133 is connected with the second end of the hollow channel 1131.

In this embodiment, the host 11 further comprises a communication tube 1134 having two ends. A first end of the communication tube 1134 is sheathed around the first hollow post 1132. Consequently, the communication tube 1134 is connected with the first hollow post 1132.

The connecting module 12 is detachably connected between the host 11 and the breathing tube 13. In this embodiment, the connecting module 12 comprises a second shell 120, a second airflow channel 121, a filter 122 and an airflow guiding part 123. The second airflow channel 121 is disposed within the connecting module 12 and aligned with the first airflow channel 112. Moreover, the second airflow channel 121 is defined by at least one part of the second shell 120. When the connecting module 12 is connected between the host 11 and the breathing tube 13, the second airflow channel 121 is in communication with the first airflow channel 112 and the breathing tube 13 so as to receive the airflow 130 from the breathing tube 13. The filter 122 is installed in the second airflow channel 121 and partially locked within the second shell 120 so as to filter the airflow 130 from the breathing tube 13. By the filter 122, the second airflow channel 121 is divided into an inlet zone 124 and an outlet zone 125. The inlet zone 124 is arranged between the filter 122 and the breathing tube 13. The outlet zone 125 is arranged between the filter 122 and the host 11. After the airflow 130 introduced into the inlet zone 124 is filtered by the filter 122, the filtered airflow 130 is exited from the outlet zone 125. The airflow guiding part 123 runs through the second shell 120 of the connecting module 12. A first end and a second end of the airflow guiding part 123 are formed as an outlet opening 1231 and an inlet opening 1232, respectively. The outlet opening 1231 is formed in an outer wall 1200 of the second shell 120 and aligned with the second hollow post 1133. The size of the outlet opening 1231 matches the size of the second hollow post 1133. When the host 11 and the connecting module 12 are combined together, the second hollow post 1133 is inserted into the outlet opening 1231 and connected with the outlet opening 1231. The inlet opening 1232 of the airflow guiding part 123 is in communication with the inlet zone 124. The airflow 130 coming from the inlet zone 124 and unfiltered by the filter 122 is received by the inlet opening 1232.

The pressure sensor 116 is installed in the host 11 and connected with a second end of the communication tube 1134. The pressure sensor 116 is used for detecting the pressure level of the received airflow. It is noted that numerous modifications and alterations may be made while retaining the teachings of the invention. For example, in another embodiment, the pressure sensor 116 is directly connected with the first hollow post 1132. Under this circumstance, the communication tube 1134 may be omitted.

Please refer to FIGS. 1 and 2 again. The first shell 111 of the host 11 further comprises a first coupling part 1121. The first coupling part 1121 is extended externally from the outer wall 115 for covering the first airflow channel 112. The connecting module 12 comprises a receiving part 1211. The receiving part 1211 is aligned with the first coupling part 1121 and located beside the outlet zone 125. Moreover, the receiving part 1211 is concaved in the second shell 120 and in communication with the outlet zone 125 of the second airflow channel 121. The size of the receiving space of the receiving part 1211 matches the size of the first coupling part 1121. When the connecting module 12 and the host 11 are combined together, the first coupling part 1121 is accommodated and locked within the receiving part 1211. Consequently, the connecting module 12 and the host 11 are in an assembled status. In this embodiment, the hollow channel 1131 runs through the first coupling part 1121, and the second hollow post 1133 is formed on a surface of the first coupling part 1121 and aligned with the outlet opening 1231.

Moreover, the connecting module 12 further comprises a second coupling part 127. The second coupling part 127 is protruded externally from the second shell 120 and located beside the inlet zone 124 for covering the inlet zone 124 of the second airflow channel 121. When the breathing tube 13 is sheathed around the second coupling part 127, the breathing tube 13 and the connecting module 12 are combined together.

Please refer to FIG. 2. When the host 11, the connecting module 12 and the breathing tube 13 are combined together, the first coupling part 1121 of the host 11 is accommodated and locked within the receiving part 1211, the second hollow post 1133 is inserted into the outlet opening 1231 and connected with the outlet opening 1231, and the breathing tube 13 is sheathed around the second coupling part 127 of the connecting module 12. Consequently, the airflow 130 from the breathing tube 13 is sequentially introduced into the inlet zone 124 of the second airflow channel 121, filtered by the filter 122, and transmitted to the host 11 through the outlet zone 125 and the first airflow channel 112. According to the airflow 130, the host 11 performs the corresponding action. As mentioned above, the pressure sensor 116 is connected with the communication tube 1134, the communication tube 1134 is in communication with the first hollow post 1132 of the communication part 113, the second hollow post 1133 of the communication part 113 is in communication with the outlet opening 1231 of the airflow guiding part 123, and the inlet opening 1232 of the airflow guiding part 123 is in communication with the inlet zone 124 for receiving the airflow 130 from the breathing tube 13. Consequently, while the airflow 130 from the breathing tube 13 is introduced into the host 11 through the second airflow channel 121 and the first airflow channel 112, the pressure level of the airflow at the inlet zone 124 is detected by the pressure sensor 116 through the communication tube 1134, the communication part 113 and the airflow guiding part 123. As previously described, the pressure sensor of the conventional respiratory device can only detect the pressure of the respiratory airflow that is produced by the user and filtered by the filter. In comparison with the pressure sensor of the conventional respiratory device, the detecting result of the pressure sensor can actually reflect the respiratory airflow pressure that is produced by the user. According to the detecting result of the pressure sensor, the respiratory device provides accurate and continuous pressure level to the user.

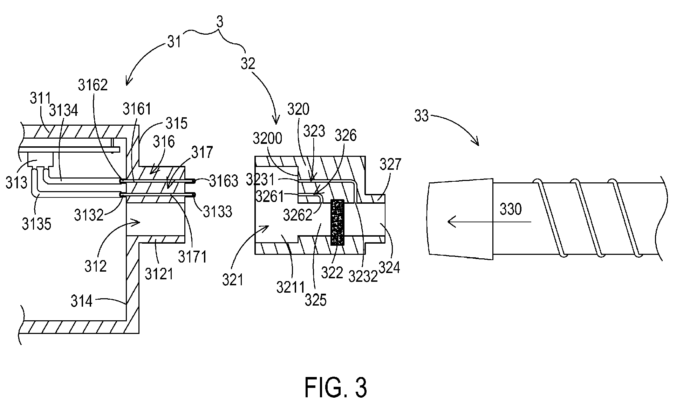

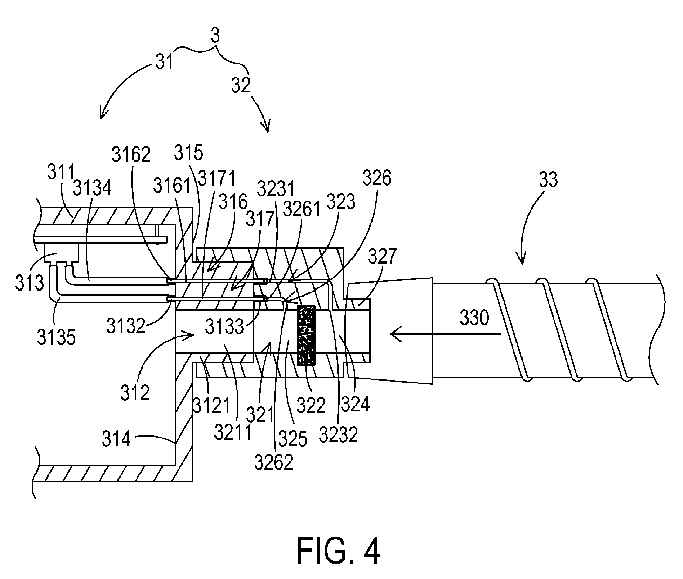

FIG. 3 is a schematic exploded view illustrating the relationship between a respiratory device and a breathing tube according to a second embodiment of the present invention. FIG. 4 is a schematic assembled view illustrating the relationship between the respiratory device and the breathing tube of FIG. 3. The respiratory device 3 is detachably connected with a breathing tube 33. After the respiratory device 3 is connected with the breathing tube 33, the respiratory device 3 and the breathing tube 33 are in communication with each other. The breathing tube 33 may receive an airflow 330, which is blown by the user. Consequently, the airflow 330 may be transmitted to the respiratory device 3 through the breathing tube 33. According to the airflow 330, the respiratory device 3 performs the corresponding actions. In this embodiment, the respiratory device 3 comprises a host 31, a connecting module 32 and a flow sensor 313. The host 31 is used for controlling the actions of the respiratory device 3. The host 31 comprises a first shell 311 and a first airflow channel 312. The first shell 311 comprises an inner wall 314, an outer wall 315, a first communication part 316 and a second communication part 317. The inner wall 314 and the outer wall 315 are opposed to each other. The first communication part 316 and the second communication part 317 run through the first shell 311. Consequently, the inner wall 314 and the outer wall 315 are connected with each other through the first communication part 316 and the second communication part 317. The first airflow channel 312 is defined by at least one part of the first shell 311.

In this embodiment, the first communication part 316 comprises a first hollow channel 3161, a first hollow post 3162 and a second hollow post 3163. The first hollow channel 3161 runs through the first shell 311, and is arranged between the inner wall 314 and the outer wall 315 of the first shell 311. A first end and a second end of the first hollow channel 3161 are connected with the inner wall 314 and the outer wall 315 of the first shell 311, respectively. The first hollow post 3162 is a hollow structure protruded from the inner wall 314 of the first shell 311. In addition, the first hollow post 3162 is connected with the first end of the first hollow channel 3161. The second hollow post 3163 is a hollow structure protruded from the outer wall 315 of the first shell 311. In addition, the second hollow post 3163 is connected with the second end of the first hollow channel 3161. The second communication part 317 comprises a second hollow channel 3171, a third hollow post 3132 and a fourth hollow post 3133. The second hollow channel 3171 runs through the first shell 311, and is arranged between the inner wall 314 and the outer wall 315 of the first shell 311. A first end and a second end of the second hollow channel 3171 are connected with the inner wall 314 and the outer wall 315 of the first shell 311, respectively. The third hollow post 3132 is a hollow structure protruded from the inner wall 314 of the first shell 311. In addition, the third hollow post 3132 is connected with the first end of the second hollow channel 3171. The fourth hollow post 3133 is a hollow structure protruded from the outer wall 315 of the first shell 311. In addition, the fourth hollow post 3133 is connected with the second end of the second hollow channel 3171.

The connecting module 32 is detachably connected between the host 31 and the breathing tube 33. In this embodiment, the connecting module 32 comprises a second shell 320, a second airflow channel 321, a filter 322, a first airflow guiding part 323 and a second airflow guiding part 326. The second airflow channel 321 is disposed within the connecting module 32 and aligned with the first airflow channel 312. Moreover, the second airflow channel 321 is defined by at least one part of the second shell 320. When the connecting module 32 is connected between the host 31 and the breathing tube 33, the second airflow channel 321 is in communication with the first airflow channel 312 and the breathing tube 33 so as to receive the airflow 330 from the breathing tube 33. The filter 322 is installed in the second airflow channel 321 and partially locked within the second shell 320 so as to filter the airflow 330 from the breathing tube 33. By the filter 322, the second airflow channel 321 is divided into an inlet zone 324 and an outlet zone 325. The inlet zone 324 is arranged between the filter 322 and the breathing tube 33. The outlet zone 325 is arranged between the filter 322 and the host 31. After the airflow 330 introduced into the inlet zone 324 is filtered by the filter 322, the filtered airflow 330 is exited from the outlet zone 325. The first airflow guiding part 323 and the second airflow guiding part 326 run through the second shell 320 of the connecting module 32. A first end and a second end of the first airflow guiding part 323 are formed as a first outlet opening 3231 and a first inlet opening 3232, respectively. A first end and a second end of the second airflow guiding part 326 are formed as a second outlet opening 3261 and a second inlet opening 3262, respectively. The first outlet opening 3231 is formed in an outer wall 3200 of the second shell 320 and aligned with the second hollow post 3163. The size of the first outlet opening 3231 matches the size of the second hollow post 3163. The second outlet opening 3261 is formed in the outer wall 3200 of the second shell 320 and aligned with the fourth hollow post 3133. The size of the second outlet opening 3261 matches the size of the fourth hollow post 3133. When the host 31 and the connecting module 32 are combined together, the second hollow post 3163 is inserted into the first outlet opening 3231 and connected with the first outlet opening 3231, and the fourth hollow post 3133 is inserted into the second outlet opening 3261 and connected with the second outlet opening 3261. The first inlet opening 3232 of the first airflow guiding part 323 is in communication with the inlet zone 324. The airflow 330 coming from the inlet zone 324 and unfiltered by the filter 322 is received by the first inlet opening 3232. The second inlet opening 3262 of the second airflow guiding part 326 is in communication with the outlet zone 325. The airflow 330 coming from the outlet zone 325 and filtered by the filter 322 is received by the second inlet opening 3262.

The host 31 further comprises a first communication tube 3134 and a second communication tube 3135. Each of the first communication tube 3134 and the second communication tube 3135 has two ends. A first end of the first communication tube 3134 is sheathed around the first hollow post 3162, so that the first communication tube 3134 is connected with the first hollow post 3162. A first end of the second communication tube 3135 is sheathed around the third hollow post 3132, so that the second communication tube 3135 is connected with the third hollow post 3132.

The flow sensor 313 is installed in the host 31, and connected with a second end of the first communication tube 3134 and a second end of the second communication tube 3135. The flow sensor 313 is used for detecting a first pressure level of the unfiltered airflow 330 corresponding to the inlet zone 324 and a second pressure level of the filtered airflow 330 corresponding to the outlet zone 325, calculating a pressure difference between the first pressure level and the second pressure level and obtaining a flow rate of the airflow 330 according to the pressure difference. It is noted that numerous modifications and alterations may be made while retaining the teachings of the invention. For example, in another embodiment, the flow sensor 313 is directly connected with the first hollow post 3162 and the third hollow post 3132. Under this circumstance, the first communication tube 3134 and the second communication tube 3135 may be omitted.

Please refer to FIGS. 3 and 4 again. The first shell 311 of the host 31 further comprises a first coupling part 3121. The first coupling part 3121 is extended externally from the outer wall 315 for covering the first airflow channel 312. The connecting module 32 comprises a receiving part 3211. The receiving part 3211 is aligned with the first coupling part 3121 and located beside the outlet zone 325. Moreover, the receiving part 3211 is concaved in the second shell 320 and in communication with the outlet zone 325 of the second airflow channel 321. The size of the receiving space of the receiving part 3211 matches the size of the first coupling part 3121. When the connecting module 32 and the host 31 are combined together, the first coupling part 3121 is accommodated and locked within the receiving part 3211. Consequently, the connecting module 32 and the host 31 are in an assembled status.

Moreover, the connecting module 32 further comprises a second coupling part 327. The second coupling part 327 is protruded externally from the second shell 320 and located beside the inlet zone 324 for covering the inlet zone 324 of the second airflow channel 321. When the breathing tube 33 is sheathed around the second coupling part 327, the breathing tube 33 and the connecting module 32 are combined together.

As shown in FIG. 4, the host 31, the connecting module 32 and the breathing tube 33 are combined together. Meanwhile, the second hollow post 3163 is inserted into the first outlet opening 3231 and connected with the first outlet opening 3231, the fourth hollow post 3133 is inserted into the second outlet opening 3261 and connected with the second outlet opening 3261, and the breathing tube 33 is sheathed around the second coupling part 327 of the connecting module 32. Consequently, the airflow 330 from the breathing tube 33 is sequentially introduced into the inlet zone 324 of the second airflow channel 321, filtered by the filter 322, and transmitted to the host 31 through the outlet zone 325 and the first airflow channel 312. According to the airflow 330, the host 31 performs the corresponding action. As mentioned above, the flow sensor 313 is connected with the first communication tube 3134 and the second communication tube 3135. Moreover, the first communication tube 3134 is in communication with the first hollow post 3162 of the first communication part 316, the second hollow post 3163 of the first communication part 316 is in communication with the first outlet opening 3231 of the first airflow guiding part 323, and the first inlet opening 3232 of the first airflow guiding part 323 is in communication with the inlet zone 324 for receiving the airflow 330 from the breathing tube 33. Moreover, the second communication tube 3135 is in communication with the third hollow post 3132, the fourth hollow post 3133 of the second communication part 317 is in communication with the second outlet opening 3261 of the second airflow guiding part 326, and the second inlet opening 3262 of the second airflow guiding part 326 is in communication with the outlet zone 325 for receiving the airflow 330 from the breathing tube 33. Consequently, while the airflow 330 from the breathing tube 33 is introduced into the host 31 through the second airflow channel 321 and the first airflow channel 312, the first pressure level of the airflow at the inlet zone 324 is detected by the flow sensor 313 through the first communication tube 3134, the first communication part 316 and the first airflow guiding part 323, and the second pressure level of the airflow at the outlet zone 325 is detected by the flow sensor 313 through the second communication tube 3135, the second communication part 317 and the second airflow guiding part 326. According to a pressure difference between the first pressure level and the second pressure level, the flow rate of the airflow 330 is calculated by the respiratory device 3. Under this circumstance, the filter 322 of the respiratory device 3 can replace the flow resistor of the conventional respiratory device. Since the respiratory device 33 of the present invention is not equipped with the flow resistor, the respiratory device 3 of the present invention is more cost-effective.

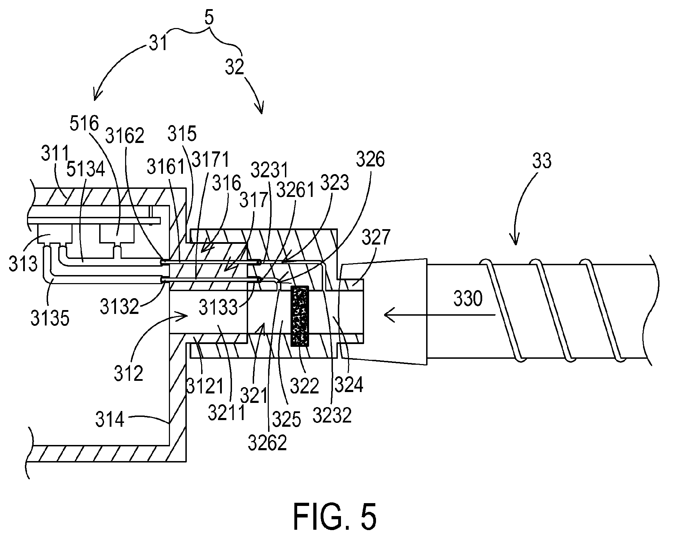

FIG. 5 is a schematic exploded view illustrating the relationship between a respiratory device and a breathing tube according to a third embodiment of the present invention. Component parts and elements corresponding to those of the second embodiment are designated by identical numeral references, and detailed descriptions thereof are omitted.

In comparison with the respiratory device 3 of the second embodiment, the respiratory device 5 of this embodiment further comprises a pressure sensor 516 for detecting a pressure level of the airflow 330. Moreover, the first communication tube 5134 comprises three ends. A first end of the first communication tube 5134 is sheathed around the first hollow post 3162, a second end of the first communication tube 5134 is in communication with the flow sensor 313, and a third end of the first communication tube 5134 is in communication with the pressure sensor 516. Consequently, both of the pressure sensor 516 and the flow sensor 313 are in communication with the inlet zone 324 through the first communication tube 5134, the first hollow post 3162, the first hollow channel 3161, the second hollow post 3163 and the first airflow guiding part 323. In other words, the pressure level of the airflow corresponding to the inlet zone 324 can be detected by the pressure sensor 516 and the flow sensor 313. In comparison with the conventional respiratory device, the detecting results of the pressure sensor 516 and the flow sensor 313 can actually reflect the respiratory airflow pressure that is produced by the user. According to the detecting results of the pressure sensor 516 and the flow sensor 313, the respiratory device 5 provides accurate and continuous pressure level to the user.

From the above descriptions, the present invention provides a respiratory device having a function of detecting an airflow pressure difference. The respiratory device includes a host, a pressure sensor and a connecting module. The host comprises a communication part. The connecting module comprises an airflow guiding part. A first end of the communication part is in communication with the pressure sensor, which is disposed within the host. A second end of the communication part is in communication with a first end of the airflow guiding part. An unfiltered airflow coming from a breathing tube is received by a second end of the airflow guiding part. Consequently, the pressure of the respiratory airflow produced by the user can be directly detected by the pressure sensor through the communication part and the airflow guiding part. In comparison with the conventional respiratory device, the detecting result of the pressure sensor of the inventive respiratory device can actually reflect the respiratory airflow pressure that is produced by the user. According to the detecting result of the pressure sensor, the respiratory device provides accurate and continuous pressure level to the user. Moreover, the pressure difference between the inlet zone and the outlet zone at opposite sides of the filter can be directly detected by the flow sensor of the respiratory device of the present invention. Consequently, the filter has the function similar to the flow resistor. Since the respiratory device of the present invention is not equipped with the flow resistor, the respiratory device of the present invention is more cost-effective.

## Claims

1. A respiratory device (1) in communication with a breathing tube (13), the respiratory device (1) comprising:
a host (11) comprising a first airflow channel (112) and a first shell (111), wherein the first shell (111) comprises a communication part (113), and the communication part (113) runs through the first shell (111);
a pressure sensor (116) installed in the host (11) and connected with the communication part (113); and
a connecting module (12) detachably connected between the host (11) and the breathing tube (13), and comprising a second airflow channel (121), a filter (122) and an airflow guiding part (123), wherein the second airflow channel (121) is in communication with the first airflow channel (112), and the filter (122) is installed in the second airflow channel (121), wherein when the host (11) and the connecting module (12) are combined together, a first end of the airflow guiding part (123) is in communication with the communication part (113), and a first portion of an airflow (130) unfiltered by the filter (122) is introduced into a second end of the airflow guiding part (123),
wherein a second portion of the airflow (130) is introduced into the host (11) through the second airflow channel (121), the filter (122) and the first airflow channel (112), and a pressure level of the unfiltered airflow (130) is detected by the pressure sensor (116) through the communication part (113) and the airflow guiding part (123).

2. The respiratory device according to claim 1, wherein the second airflow channel (121) is divided into an inlet zone (124) and an outlet zone (125) by the filter (122), wherein the inlet zone (124) is arranged between the filter (122) and the breathing tube (13), and the outlet zone (125) is arranged between the filter (122) and the host (11), wherein the second portion of the airflow (130) is transmitted through the inlet zone (124) and the filter (122) and exited from the outlet zone (125).

3. The respiratory device according to claim 2, wherein the first shell (111) further comprises an inner wall (114) and an outer wall (115), wherein the inner wall (114) and the outer wall (115) are opposed to each other, and the inner wall (114) and the outer wall (115) are connected with each other through the communication part (113), wherein the communication part (113) comprises a hollow channel (1131), a first hollow post (1132) and a second hollow post (1133), wherein the hollow channel (1131) runs through the first shell (111), and is arranged between the inner wall (114) and the outer wall (115) of the first shell (111), wherein the hollow channel (1131) is connected between the first hollow post (1132) and the second hollow post (1133), the first hollow post (1132) is protruded from the inner wall (114) of the first shell (111), and the second hollow post (1133) is protruded from the outer wall (115) of the first shell (111).

4. The respiratory device according to claim 3, wherein the airflow guiding part (123) is disposed within a second shell (120) of the connecting module (12), the first end of the airflow guiding part (123) has an outlet opening (1231), and the second end of the airflow guiding part (123) has an inlet opening (1232), wherein the outlet opening (1231) is formed in an outer wall (1200) of the second shell (120) and aligned with the second hollow post (1133), wherein when the host (11) and the connecting module (12) are combined together, the second hollow post (1133) is inserted into the outlet opening (1231) and connected with the outlet opening (1231), wherein the inlet opening (1231) of the airflow guiding part (123) is in communication with the inlet zone (124).

5. The respiratory device according to claim 4, wherein the connecting module (12) further comprises a second coupling part (127), wherein the second coupling part (127) is protruded externally from the second shell (120) and located beside the inlet zone (124) for covering a part of the second airflow channel (121), wherein the breathing tube (13) is sheathed around the second coupling part (127).

6. The respiratory device according to claim 4, wherein the first shell (111) further comprises a first coupling part (1121), wherein the first coupling part (1121) is extended externally from the outer wall (115) of the first shell (111) for covering the first airflow channel (112), wherein the hollow channel (1131) runs through the first coupling part (1121), and the second hollow post (1133) is formed on a surface of the first coupling part (1121), wherein the connecting module (12) comprises a receiving part (1211), wherein the receiving part (1211) is aligned with the first coupling part (1121) and located beside the outlet zone (125), and the receiving part (1211) is concaved in the second shell (120) and in communication with the outlet zone (125) of the second airflow channel (121), wherein when the connecting module (12) and the host (11) are combined together, the first coupling part (1121) is accommodated and locked within the receiving part (1211).

7. The respiratory device according to claim 3, wherein the host (11) further comprises a communication tube (1134), wherein a first end of the communication tube (1134) is sheathed around the first hollow post (1132) and connected with the first hollow post (1132), and a second end of the communication tube (1134) is in communication with the pressure sensor (116).

8. A respiratory device (5) in communication with a breathing tube (33), the respiratory device (5) comprising:
a host (31) comprising a first airflow channel (312) and a first shell (311), wherein the first shell (311) comprises a first communication part (316) and a second communication part (317), wherein the first communication part (316) and the second communication part (317) run through the first shell (311);
a flow sensor (313) installed in the host (31), and connected with the first communication part (316) and the second communication part (317);
a pressure sensor (516) installed in the host (31) and connected with the first communication part (316); and
a connecting module (32) detachably connected between the host (31) and the breathing tube (33), and comprising a second airflow channel (321), a filter (322), a first airflow guiding part (323) and a second airflow guiding part (326), wherein the second airflow channel (321) is in communication with the first airflow channel (312), and the filter (322) is installed in the second airflow channel (321), wherein when the host (31) and the connecting module (32) are combined together, a first end of the first airflow guiding part (323) is in communication with the first communication part (316), a first end of the second airflow guiding part (326) is in communication with the second communication part (317), a first portion of an airflow (330) unfiltered by the filter (322) is introduced into a second end of the first airflow guiding part (323), and a second portion of the airflow (330) filtered by the filter (322) is introduced into a second end of the second airflow guiding part (326),
wherein a third portion of the airflow (330) is introduced into the host (31) through the second airflow channel (321), the filter (322) and the first airflow channel (312), wherein a first pressure level of the unfiltered airflow is detected by both of the flow sensor (313) and the pressure sensor (516) through the first communication part (316) and the first airflow guiding part (323), a second pressure level of the filtered airflow is detected by the flow sensor (313) through the second communication part (317) and the second airflow guiding part (326), and a pressure difference between the first pressure level and the second pressure level is obtained by the flow sensor (313).

9. A respiratory device (1) in communication with a breathing tube (13), the respiratory device (1) comprising:
a host (11) comprising a first airflow channel (112) and a first shell (111), wherein the first shell (111) comprises a communication part (113), and the communication part (113) runs through the first shell (111);
a pressure sensor (116) installed in the host (11) and connected with the communication part (113); and
a connecting module (12) detachably connected between the host (11) and the breathing tube (13), and comprising a second airflow channel (121), a filter (122) and an airflow guiding part (123), wherein the second airflow channel (121) is in communication with the first airflow channel (112), the airflow guiding part (123) is arranged between the filter (122) and the breathing tube (13), and the filter (122) is installed in the second airflow channel (121), wherein when the host (11) and the connecting module (12) are combined together, the communication part (113) is in communication with the airflow guiding part (123) so as to receive an airflow (130) from the breathing tube (13).

10. The respiratory device according to claim 9, wherein when the host (11) and the connecting module (12) are combined together, a first end of the airflow guiding part (123) is in communication with the communication part (113), and a first portion of the airflow (130) unfiltered by the filter (122) is introduced into a second end of the airflow guiding part (123).

11. The respiratory device according to claim 10, wherein the communication part (113) comprises a hollow channel (1131), a first hollow post (1132) and a second hollow post (1133), wherein the hollow channel (1131) runs through the first shell (111), and is arranged between an inner wall (114) and an outer wall (115) of the first shell (111), wherein the hollow channel (1131) is connected between the first hollow post (1132) and the second hollow post (1133), the first hollow post (1132) is protruded from the inner wall (114) of the first shell (111), and the second hollow post (1133) is protruded from the outer wall (115) of the first shell (111), wherein the first end of the airflow guiding part (123) has an outlet opening (1231), and the second end of the airflow guiding part (123) has an inlet opening (1232), wherein the outlet opening (1231) is formed in an outer wall (1200) of a second shell (120) of the connecting module (12) and aligned with the second hollow post (1133), wherein when the host (11) and the connecting module (12) are combined together, the second hollow post (1133) is inserted into the outlet opening (1231) and connected with the outlet opening (1231), wherein the inlet opening (1232) of the airflow guiding part (123) is in communication with a zone between the filter (122) and the breathing tube (13).

12. A respiratory device (3) in communication with a breathing tube (33), the respiratory device (3) comprising:
a host (31) comprising a first airflow channel (312) and a first shell (311), wherein the first shell (311) comprises a first communication part (316) and a second communication part (317), wherein the first communication part (316) and the second communication part (317) run through the first shell (311), respectively;
a flow sensor (313) installed in the host (31), and connected with the first communication part (316) and the second communication part (317); and
a connecting module (32) detachably connected between the host (31) and the breathing tube (33), and comprising a second airflow channel (321), a filter (322), a first airflow guiding part (323) and a second airflow guiding part (326), wherein the second airflow channel (321) is in communication with the first airflow channel (312), the filter (322) is installed in the second airflow channel (321), and the first airflow guiding part (323) is arranged between the filter (322) and the breathing tube (33), wherein when the host (31) and the connecting module (32) are combined together, the first airflow guiding part (323) is in communication with the first communication part (316), the second airflow guiding part (326) is in communication with the second communication part (317), and the first airflow guiding part (323) and the second airflow guiding part (326) receive an airflow (330) from the breathing tube (33) and provide the airflow (330) to the flow sensor (313), respectively, so that a pressure difference of the airflow (330) is obtained.

13. The respiratory device according to claim 12, wherein when the host (31) and the connecting module (32) are combined together, a first end of the first airflow guiding part (323) is in communication with the first communication part (316), a first end of the second airflow guiding part (326) is in communication with the second communication part (317), a first portion of the airflow (330) unfiltered by the filter (322) is introduced into a second end of the first airflow guiding part (323), and a second portion of the airflow (330) filtered by the filter (322) is introduced into a second end of the second airflow guiding part (326).

14. The respiratory device according to claim 13, wherein the first communication part (316) comprises a first hollow channel (3161), a first hollow post (3162) and a second hollow post (3163), and the second communication part (317) comprises a second hollow channel (3171), a third hollow post (3132) and a fourth hollow post (3133), wherein the first hollow channel (3161) and the second hollow channel (3171) run through the first shell (311), and are arranged between an inner wall (314) and an outer wall (315) of the first shell (311), respectively, wherein the first hollow channel (3161) is connected between the first hollow post (3162) and the second hollow post (3163), and the second hollow channel (3171) is connected between the third hollow post (3132) and the fourth hollow post (3133), wherein the first hollow post (3162) is protruded from the inner wall (314) of the first shell (311), the second hollow post (3163) is protruded from the outer wall (315) of the first shell (311), the third hollow post (3132) is protruded from the inner wall (314) of the first shell (311), and the fourth hollow post (3133) is protruded from the outer wall (315) of the first shell (311).

15. The respiratory device according to claim 14, wherein the first end of the first airflow guiding part (323) has a first outlet opening (3231), the second end of the first airflow guiding part (323) has a first inlet opening (3232), the first end of the second airflow guiding part (326) has a second outlet opening (3261), and the second end of the second airflow guiding part (326) has a second inlet opening (3262), wherein the first outlet opening (3231) is formed in an outer wall (3200) of a second shell (320) of the connecting module (32) and aligned with the second hollow post (3163), and the second outlet opening (3261) is formed in the outer wall (3200) of the second shell (320) of the connecting module (32) and aligned with the fourth hollow post (3133), wherein when the host (31) and the connecting module (32) are combined together, the second hollow post (3163) is inserted into the first outlet opening (3231) and connected with the first outlet opening (3231), and the fourth hollow post (3133) is inserted into the second outlet opening (3261) and connected with the second outlet opening (3261), wherein the first inlet opening (3231) of the first airflow guiding part (323) is in communication with a zone between the filter (322) and the breathing tube (33), and the second inlet opening (3261) of the second airflow guiding part (326) is in communication with a zone between the filter (322) and the host (31).
